# EUROPEAN PATENT APPLICATION

(11) **EP 4 137 050 A1**
(43) Date of publication of application: **22.02.2023**
(21) Application number: 21788435.2
(22) Date of filing: 13.04.2021
(51) Int. Cl.: A61B 5/266, A61B 5/273

(54) **BIOELECTRODE THAT CAN BE WORN FOR A LONG PERIOD OF TIME**

(30) Priority: 14.04.2020 JP 2020072499
(71) Applicant: I Medex Co., Ltd., Chiba-shi, Chiba 262-0003 (JP)
(72) Inventor: ICHIDA Shinshichi, Chiba-shi Chiba 262-0003 (JP); ICHIDA Makoto, Chiba-shi Chiba 262-0003 (JP); MINOWA Takashiro, Chiba-shi Chiba 262-0003 (JP); NAGAHAMA Norio, Chiba-shi Chiba 262-0003 (JP)
(74) Representative: Lavoix
(86) International application number: PCT/JP2021/015362
(87) International publication number: WO 2021/210592

(57) **Abstract**

A bioelectrode is provided that is formed such that, as a bioelectrode that prevents swelling of an electrolyte in the electrode portion, does not make measurement unstable or impossible due to falling off of the electrolyte from the electrode part or a living body surface even when the bioelectrode is worn for a long time, does not cause a feeling of discomfort such as itchiness, and further prevents a decrease in adhesive force, the bioelectrode includes an electrode portion that acquires an electric signal of a living body or outputs an electric signal to the living body, in which the electrode portion is provided with an electrolyte layer that is in close contact with the living body, and is also provided with a sheet-like cover member that covers at least a part or all of the electrolyte layer, the cover member being provided with an opening that penetrates in a thickness direction, thereby discharging moisture accumulated in the electrolyte layer and preventing the swelling and deterioration.

## Description

### Technical Field

The present invention relates to a bioelectrode that is wearable for a long time, and more particularly, to a bioelectrode that is wearable for a long time while preventing swelling and deterioration by discharging moisture of an electrolyte layer especially in contact with a living body surface. The present invention also relates to a bioelectrode having a waterproof function that is wearable for a long time by achieving waterproofing between terminals connected to a biosignal processor.

### Background Art

In order to grasp the condition of a subject/patient, biosignals such as potential information acquired from a living body have conventionally been used. To acquire such biosignals, a Holter electrocardiograph that is wearable for a long time is used because it is sometimes necessary to perform measurement for a long time in daily life. A need exists for such a Holter electrocardiograph to allow a patient/subject wearing the Holder electrocardiograph to take a shower or take a bath. Thus, a biopotential detection device capable of measuring a biopotential (electrocardiogram) even while a patient/subject is taking a shower or taking a bath has been proposed before.

For example, Patent Literature 1 (JP 2004-121360 A) proposes a biopotential detection device including a disposable bioelectrode pad for detecting a potential of a living body and a reusable signal processor that processes a biopotential signal detected by the bioelectrode pad, in which the bioelectrode pad and the signal processor are detachable, and a waterproof structure is applied to a portion for guiding the biopotential from the bioelectrode pad to the signal processor.

In addition, in Patent Literature 2 (JP 2014-222608 A), the applicant of the present application proposes a connection structure with a connector in which a waterproofing measure can be applied to a connection portion of a flat cable with the connector, and a touch-proof structure is further achieved. That is, this literature proposes the connection structure of the flat cable with the connector that eliminates a possibility that the flat cable comes off due to an external force as well as achieving a waterproof property and reliability of electrical connection by providing a cushion sheet made of rubber or a foamed resin at the connection portion of the flat cable with the connector, and further providing the cushion sheet with contact holes for connection with a plurality of contact pins connected to core wires of a device-side cable.

### Citation List

### Patent Literature

Patent Literature 1: JP 2004-121360 A
Patent Literature 2: JP 2014-222608 A

### Summary of Invention

### Technical Problem

As described above, there has conventionally been a demand for measurement for a long time in daily life for acquisition of biosignals. In such a bioelectrode, an electrolyte such as a gel is often used for an electrode portion in order to acquire signals from a living body.

When the electrode portion is provided with a waterproof structure, water entry from the outside can be prevented, whereas sweat or the like generated from the living body cannot be released to the outside. As a result, when the bioelectrode is worn for a long time, there is a risk of deterioration such as decrease in adhesive force due to sweat, water at the time of bathing, or the like accumulated in the electrolyte such as a gel. In addition, in a case where the electrolyte is swollen and deteriorated due to sweat or the like when the bioelectrode is worn for a long time, there is a risk that the electrolyte falls off an electrode part or a living body surface to make the measurement unstable or impossible.

Therefore, an object of the present invention is to provide a bioelectrode that prevents swelling of the electrolyte in the electrode portion, that does not make the measurement unstable or impossible due to the falling off of the electrolyte from the electrode part or the living body surface even when the bioelectrode is worn for a long time, and that further prevents the decrease in adhesive force.

In addition, even in a case where the electrolyte is swollen, as long as its outflow or deformation can be prevented, the measurement does not become unstable or impossible due to the falling off of the electrolyte from the electrode part or the living body surface even when the bioelectrode is worn for a long time, and it is further possible to prevent the decrease in adhesive force. Therefore, another object of the present invention is to provide a bioelectrode capable of preventing the outflow and deformation of the electrolyte even if the electrolyte is swollen.

Furthermore, a waterproof function has conventionally been required in consideration of scenes such as exercise and bathing during the long-time wearing. Thus, it has been proposed that the waterproof structure is provided to the biopotential guiding portion in Patent Literature 1. However, the waterproof property of the signal processor and the problems of the electrode portion during the long-time wearing have not been studied at all.

Therefore, another object of the present invention is to provide a bioelectrode that is wearable for a long time by studying the signal processing device and the electrode portion.

In addition, as described above, Patent Literature 1 proposes the biopotential detection device in which the portion for guiding the biopotential from the bioelectrode pad to the signal processor is provided with the waterproof structure. This technology is based on the premise that the signal processing device has a waterproof structure. That is, a signal processing device having no waterproof function cannot be used.

Therefore, another object of the present invention is to provide a bioelectrode that is wearable for a long time, including bathing or the like, not only when the biosignal processing device has a waterproof property but also when the biosignal processing device does not have a waterproof structure.

### Solution to Problem

In order to achieve at least any one of the above objects, the present invention provides a bioelectrode capable of preventing swelling and deterioration of an electrolyte layer by studying an electrode portion so as to provide a bioelectrode that is wearable for a long time.

That is, the present invention provides a bioelectrode including an electrode portion that acquires an electric signal of a living body or outputs an electric signal to the living body, in which the electrode portion is provided with an electrolyte layer that is in close contact with the living body, the electrode portion is also provided with a sheet-like cover member that covers at least a part or all of a surface of the electrolyte layer on a side not in close contact with the living body, and an opening that penetrates in a thickness direction is provided in a region of the cover member covering the electrolyte layer.

The sheet-like cover member can function to hold the electrolyte layer at a predetermined position, and can also function to hold at least the electrode portion on the living body. The cover member can be formed using a sheet having a waterproof property, and a tape having a pressure-sensitive adhesive or adhesive layer can be used. As an example, a tape made of a resin film or a fabric (including a cloth and a nonwoven fabric) can be used. Therefore, the bioelectrode can be stuck or attached to the living body by the cover member.

In addition, the cover member is provided with the opening penetrating in the thickness direction. This opening is formed in the region of the cover member covering the electrolyte layer, and desirably has a function as a check valve that releases moisture of the covered electrolyte layer to the outside and/or prevents water entry from the outside. That is, the opening is a fine hole capable of releasing moisture as a liquid or a gas, or may also be formed as a large opening unless the covered electrolyte leaks.

From the above description, the bioelectrode according to the present invention can be provided as a bioelectrode that acquires an electric signal of a living body or outputs an electric signal to the living body, the bioelectrode including an electrode portion used in close contact with the living body, in which the electrode portion includes an electrolyte layer that is in close contact with the living body and a cover member that sticks the electrode layer to the living body, and the cover member is provided with an opening for discharging moisture of the electrolyte layer.

Even in a case where the electrolyte layer is swollen due to sweat generated from the living body, moisture entering from an external environment, or the like, according to the bioelectrode of the present invention, when the electrolyte is provided on both sides in a thickness direction of a substrate, and a hydrophilic gel is used for its upper layer, there is a waterproof effect using the characteristic that the electrolyte does not absorb moisture any more when the moisture absorption amount reaches a saturated state. Furthermore, the two-layer gel structure acts to constantly equalize the water contents of the two layers. As a result, it is possible to release moisture from the opening to the outside, thereby avoiding the deterioration of the electrolyte layer. Therefore, the bioelectrode can solve the problem of a decrease in adhesive force due to the swelling and deterioration of the electrolyte layer, and does not make measurement unstable or impossible due to falling off of the electrolyte layer from the electrode part or the living body surface even when the bioelectrode is worn for a long time.

In addition, in order to achieve at least any one of the above objects, the present invention provides a bioelectrode including an electrode portion that acquires an electric signal of a living body or outputs an electric signal to the living body, in which the electrode portion is formed by laminating a conductive material on a substrate, an electrolyte layer that is in close contact with the living body is provided on a surface of the substrate on a side where the conductive material is laminated, and the substrate is provided with an opening that penetrates in a thickness direction.

In some of currently available bioelectrodes, an electrode layer, a wiring layer, or the like are laminated on a substrate formed in a film shape, a sheet shape, or the like. Even in the bioelectrode using such a substrate, moisture of the electrolyte layer swollen by sweat or the like can also be released through the opening. As a result, it is possible to prevent the swelling and deterioration of the electrolyte layer such as a gel, and solve the problem that the electrolyte falls off the electrode part or the living body surface to make the measurement unstable or impossible.

Similarly to the opening provided in the cover member, the opening provided in the substrate is a fine hole capable of releasing moisture as a liquid or a gas because it is only necessary for the opening to allow excessive moisture in the electrolyte to pass, or may also be formed as a large opening unless the electrolyte leaks.

In the bioelectrode, it is also desirable that the substrate is provided with a support member that supports a rim of the electrolyte layer in a state where at least a part of the electrolyte layer is exposed to the living body side. The support member can be formed in an annular shape so as to expose a central region or the like of the electrolyte layer. In order to enhance the ability to hold the electrolyte layer, a resin sheet subjected to raising, embossing, or the like can be used in addition to a cloth such as a woven fabric, a knitted fabric, or a nonwoven fabric. The support member can be provided so as to surround a periphery of the electrolyte layer on the surface of the substrate on the side where the conductive material is laminated.

In the electrode formed using the substrate, the electrolyte may be provided on both sides in the thickness direction of the substrate. At this time, the electrolyte provided on the surface on the living body side of the substrate and the electrolyte provided on the surface on the opposite side may be different. For example, a water-absorbent gel may be used as the electrolyte provided on the surface on the living body side of the substrate, and a hydrophilic gel may be used as the electrolyte provided on the opposite side.

The opening provided in the cover member or the substrate as described above can be closed by a member having water permeability or water absorbency formed of a hydrophilic gel, a water-absorbent gel, a hydrophobic gel, a cloth, or a porous sheet, or can be openably closed by a sheet member. In particular, the cloth may be any of a woven fabric, a knitted fabric, or a nonwoven fabric. The sheet member itself may have moisture permeability or air permeability.

In the case where the opening provided in the cover member is closed by the member that absorbs water or allows water to permeate, such as a hydrophilic gel, a water-absorbent gel, a hydrophobic gel, a cloth, or a porous sheet, or is openably closed by the sheet member, it is possible to suppress outflow of the covered electrolyte layer, and it is also possible to prevent the electrolyte from adhering to clothing at the time of wearing. In the case where the opening provided in the substrate is closed by the member that absorbs water or allows water to permeate, such as a hydrophilic gel, a water-absorbent gel, a hydrophobic gel, a cloth, or a porous sheet, or is openably closed by the sheet member using the check valve function, it is possible to prevent movement of the electrolyte due to passage through the opening.

Therefore, in the bioelectrode according to the present invention, any one or a combination of the following (1) to (3) can be provided for the opening:
(1) A hydrophilic gel provided on a surface facing the opening in the electrolyte layer
(2) A cloth (including a woven fabric, a knitted fabric, or a nonwoven fabric) provided so as to close the inside of the opening in the electrolyte layer
(3) A sheet member that openably closes an upper surface of the opening, or a sheet member having moisture permeability or air permeability that opens and closes the upper surface of the opening

The hydrophilic gel, the cloth, or the sheet member provided for the opening as described above can also function as a waterproof member for preventing moisture entry into the electrolyte layer, thereby suppressing the swelling and deterioration of the electrolyte due to the moisture entry from the external environment.

The present invention also provides a bioelectrode configured such that moisture phase-transfers between a water-absorbent gel and a hydrophilic gel. That is, the bioelectrode can be provided as a bioelectrode including an electrode portion that acquires an electric signal of a living body or outputs an electric signal to the living body, in which the electrode portion is provided with an electrolyte layer that is in close contact with the living body, and at least the electrolyte layer is formed by laminating a water-absorbent gel layer formed of a gel having water absorbency and a hydrophilic gel layer formed of a gel having hydrophilicity in a part or all of a region. Such a bioelectrode can be formed with a structure having a plurality of layers in which the water-absorbent gel layer and the hydrophilic gel layer are alternately laminated, and for example, can be formed as a structure in which the hydrophilic gel layer is sandwiched between the water-absorbent gel layers. The phase transfer of moisture between the water-absorbent gel and the hydrophilic gel is achieved as described above. Thus, even if the water-absorbent gel layer excessively absorbs water, the water can be phase-transferred to the hydrophilic gel layer, so that the water content of the water-absorbent gel can be adjusted. When the water-absorbent gel layer becomes dry and lacks moisture, the water content of the water-absorbent gel layer can be adjusted by supplying the moisture of the hydrophilic gel layer to the water-absorbent gel layer.

According to the bioelectrode configured as described above, it is possible to provide the bioelectrode capable of suppressing the swelling and deterioration of the electrolyte layer and solving the problem of stuffiness (itchiness), separation, or the like at the electrode portion even when the bioelectrode is worn for a long time.

In addition, in order to achieve at least any one of the above objects, particularly to provide the bioelectrode that is wearable for a long time, the present invention provides a bioelectrode that can waterproof a biosignal processing device as necessary, and in a case where the biosignal processing device has a waterproof function, achieves waterproofing between terminals by using the biosignal processing device.

That is, the present invention provides a bioelectrode including an electrode portion that acquires an electric signal of a living body or outputs an electric signal to the living body, the bioelectrode including a terminal portion including two or more pole terminals that input or output the electric signal in the electrode portion to the outside, the bioelectrode further including a waterproof member that waterproofs at least a connection portion between the terminal portion and a terminal portion of a biosignal processor in a state where the terminal portion is connected to the terminal portion of the biosignal processor.

The biosignal processor is a device for receiving the biosignal acquired by the bioelectrode or transmitting the signal to the bioelectrode. The biosignal processor itself performs information processing of the biosignal acquired from the bioelectrode, or the biosignal processor includes a processor that acquires and stores the biosignal or transmits the signal to an external device.

Furthermore, in the bioelectrode, the waterproof member can be formed to define a housing space that houses the biosignal processor in a waterproof environment. At this time, the waterproof member can be configured to cover and house the whole biosignal processor to achieve waterproofing, or can also be configured to house at least a region where the electrode portion exists to waterproof the electrode portion. In the case where a part of the biosignal processor is housed, a waterproof structure can be achieved between the bioelectrode and the biosignal processor.

That is, the housing space formed by the waterproof member is a space portion for housing the biosignal processing device. In a case where the biosignal processing device has a waterproof function, the housing space can house the biosignal processing device in a state where a part of the biosignal processing device is exposed. On the other hand, in a case where the biosignal processing device has no waterproof function, the housing space can be formed as a waterproof space that covers and houses the whole biosignal processing device.

In addition, the bioelectrode can be formed as a bioelectrode including a waterproof member that has a waterproof structure for achieving waterproofing between input and output terminals and forms a housing space that houses a biosignal processor including a terminal connected to the output terminal.

The input and output terminals can be formed as protruding two-pole snaps penetrating the substrate, and the waterproof member can be configured by a bottomed cylindrical waterproof member main body having the housing space that houses the biosignal processor including the terminal connected to the two-pole snaps, and a lid member closing the housing space. In the waterproof member main body, a bottom portion thereof is in close contact with the substrate to prevent moisture entry, and the two-pole snaps protrude from a bottom surface into the housing space. The bioelectrode waterproofs the housing space by providing the waterproof structure on a joining surface between the waterproof member main body and the lid member. This bioelectrode is particularly effective in a case where the biosignal processing device having no waterproof function is used, and can also waterproof the biosignal processing device. However, as a matter of course, the bioelectrode can be used for the biosignal processing device having a waterproof function.

In addition, in the present invention, the input and output terminals can be formed as the protruding two-pole snaps penetrating the substrate, and the waterproof member includes the bottomed cylindrical waterproof member main body in which the bottom portion is in close contact with the substrate to achieve waterproofing and the two-pole snaps protrude from the bottom surface. The housing space that houses the biosignal processor including the terminal connected to the two-pole snaps is provided in the waterproof member main body, and the waterproof structure that is engaged with an outer surface of the biosignal processor to waterproof a region where the two-pole snaps protrude can be provided on at least any one of an inner wall surface and the bottom surface of the waterproof member main body. Particularly when the biosignal processing device has a waterproof structure, the bioelectrode can achieve waterproofing between the terminals by using the waterproof structure.

Furthermore, in the present invention, in a structure of the waterproof member, the internal space can be formed so as to be kept in a waterproof environment. That is, the waterproof member includes the bottomed cylindrical waterproof member main body in which the two-pole snaps protrude from the bottom surface, and the lid member that closes the internal space of the waterproof member main body, and waterproofing is achieved at a joining portion between the waterproof member main body and the lid member, so that waterproofing of the internal space of the waterproof member is achieved. The coupling structure between the waterproof member main body and the lid member may be a screwing structure or a joining structure in addition to a fitting structure, and a seal member such as an O-ring may be provided as necessary.

Note that the input and output terminals in the bioelectrode according to the present invention can be formed not only as the two-pole snaps but also in various shapes and structures. For example, the input and output terminals may be formed by applying the terminals to the substrate, and the terminals may be interlocked or fitted with the biosignal processor. That is, in the bioelectrode according to the present invention, the input and output terminals only need to be input and output connectors, and can be formed in combination with, for example, a waterproof connector.

In addition, in the bioelectrode including the waterproof member, the electrode portion can be formed as a bioelectrode in which the opening penetrating in the thickness direction is provided in the sheet-like cover member covering the electrolyte layer, the opening penetrating in the thickness direction is provided in the substrate on which the electrolyte layer is provided, these openings are closed by the member having water permeability or water absorbency formed of a hydrophilic gel, a water-absorbent gel, a hydrophobic gel, a cloth, or a porous sheet, or are openably closed by the sheet member, or the electrolyte layer is formed by laminating the water-absorbent gel layer formed of a gel having water absorbency and the hydrophilic gel layer formed of a gel having hydrophilicity in a part or all of the region.

### Advantageous Effects of Invention

According to the bioelectrode of the present invention, since the opening penetrating in the thickness direction is provided in the region covering the electrolyte layer in the sheet-like cover member that covers a part or all of the surface of the electrolyte layer on the side not in close contact with the living body, moisture excessively accumulated in the electrolyte layer can be released to the outside from the opening. Therefore, it is possible to provide the bioelectrode that suppresses the swelling and deterioration of the electrolyte layer in the electrode portion, and as a result, does not make the measurement unstable or impossible due to the falling off of the electrolyte layer from the electrode part or the living body surface even when the bioelectrode is worn for a long time, and further prevents the decrease in adhesive force.

Furthermore, in the bioelectrode formed using the substrate having a film shape or the like, the opening penetrating in the thickness direction is also provided in the substrate, so that a body fluid such as sweat exuded from the living body can be transferred to the upper layer (the side away from the living body) and released from the electrolyte layer to the outside. Therefore, it is possible to provide the bioelectrode that suppresses the swelling and deterioration of the electrolyte layer present closer to the living body than the substrate, and as a result, does not make the measurement unstable or impossible due to the falling off of the electrolyte layer from the electrode part or the living body surface even when the bioelectrode is worn for a long time, and further prevents the decrease in adhesive force.

In the case where the support member that supports the rim of the electrolyte layer is provided on the substrate, the support member can support the electrolyte layer swollen by absorbing moisture. Therefore, the electrolyte can be held on the electrode part or the living body surface even when the bioelectrode is worn for a long time, and the problem that the measurement becomes unstable or impossible due to the falling off of the electrolyte layer can be solved.

In addition, the bioelectrode provided with the waterproof member that waterproofs the terminal portion of the bioelectrode is wearable even during bathing, so that the bioelectrode that is wearable for a long time can be provided.

The waterproof member is configured to house the biosignal processor that acquires the signal from the bioelectrode and waterproofs the housing space. Therefore, even a biosignal processor having no waterproof structure is wearable during bathing or the like, so that the bioelectrode that is wearable for a long time can be provided.

### Brief Description of Drawings

Fig. 1(A) is an internal transparent plan view, Fig. 1(B) is a cross-sectional view taken along line B-B, and Fig. 1(C) is a cross-sectional view taken along line C-C of an electrode portion in a bioelectrode according to the present embodiment.
Fig. 2 is an exploded perspective view of the electrode portion in the bioelectrode illustrated in Fig. 1.
Fig. 3 is a longitudinal cross-sectional view of an electrode portion in a bioelectrode according to another embodiment.
Fig. 4(A) is a plan view, Fig. 4(B) is an exploded perspective view, and Fig. 4(C) is a cross-sectional view taken along line X-X of an electrode portion in a bioelectrode according to another embodiment.
Fig. 5 is a perspective view illustrating the bioelectrode using the electrode portion illustrated in Fig. 1.
Fig. 6(A) is a longitudinal cross-sectional view of the bioelectrode illustrated in Fig. 5, Fig. 6(B) is a plan view of the bioelectrode in a state where a lid member is removed, and Fig. 6(C) is a bottom view of the bioelectrode.
Fig. 7(A) is a plan view, Fig. 7(B) is a longitudinal cross-sectional view, and Fig. 7(B) is a bottom view illustrating a bioelectrode according to another embodiment.

### Description of Embodiments

Hereinafter, a bioelectrode that is wearable for a long time according to the present embodiment will be specifically described with reference to the drawings. In particular, in the present embodiment, a bioelectrode formed using a substrate 11 and further including a waterproof member 25 capable of housing the whole of a biosignal processor 50 will be specifically illustrated.

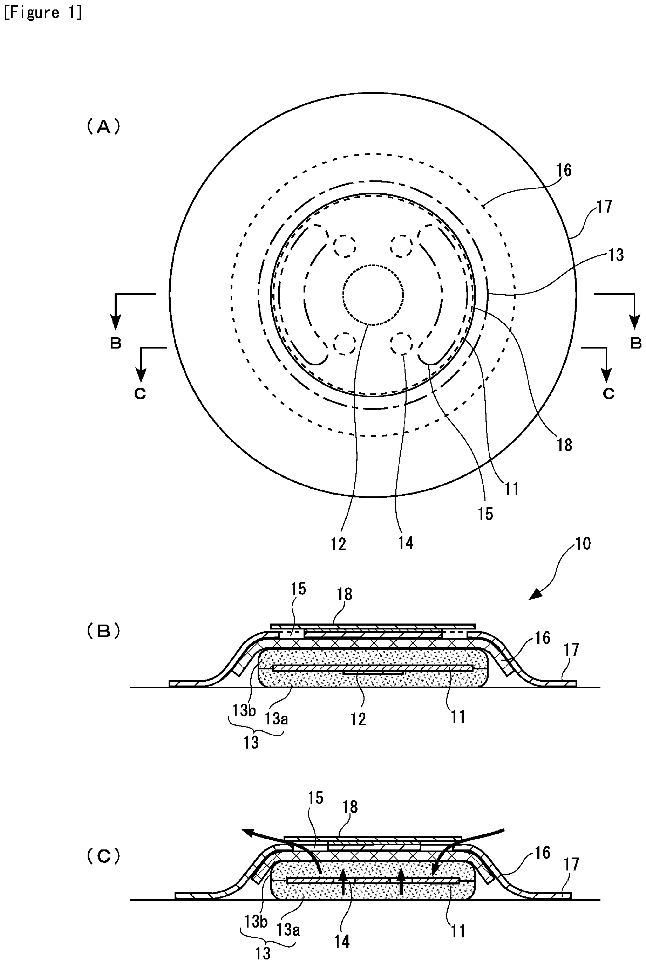

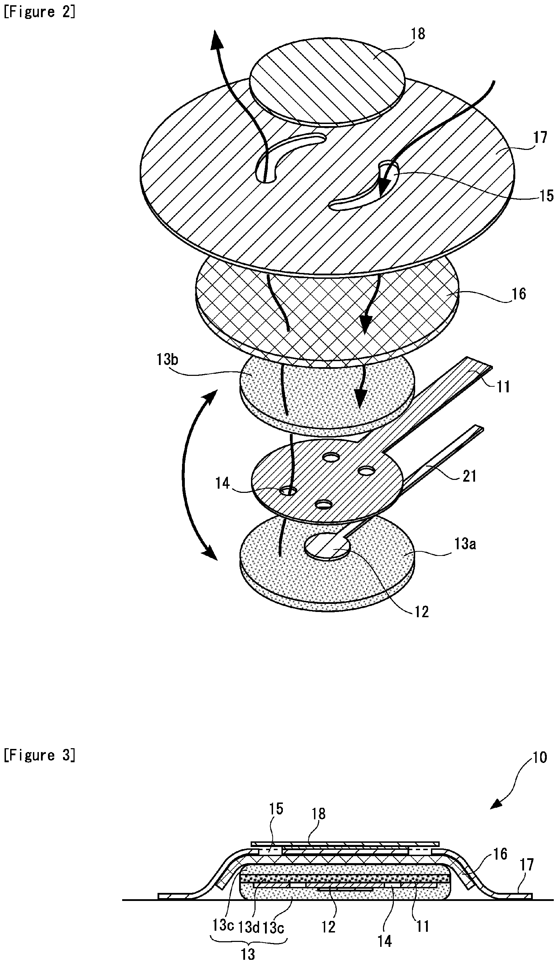

Fig. 1(A) illustrates an internal transparent plan view, Fig. 1(B) illustrates a cross-sectional view taken along line B-B, and Fig. 1(C) illustrates a cross-sectional view taken along line C-C of an electrode portion 10 in the bioelectrode according to the present embodiment. Fig. 2 illustrates an exploded perspective view of the electrode portion 10 in the bioelectrode.

As illustrated in Figs. 1 and 2, in the bioelectrode according to this embodiment, an electrode layer 12 is provided on a surface of the substrate 11 on the side of a living body, and electrolyte layers 13 (a first electrolyte layer 13a and a second electrolyte layer 13b) made of a gel are provided on the surface on the living body side and a surface on its opposite side of the substrate 11 provided with the electrode layer 12. As a result, the substrate 11 and the electrode layer 12 have a structure sandwiched between the electrolyte layers 13. In the present embodiment, the electrolyte layer 13 (hereinafter, referred to as the "first electrolyte layer 13a") made of a hydrophilic gel or a water-absorbent gel is provided on the surface on the living body side of the substrate 11 (the surface where the electrode layer 12 exists), and the electrolyte layer 13 (hereinafter, referred to as the "second electrolyte layer 13b") made of a hydrophilic gel is provided on the surface on the opposite side (hereinafter, referred to as an "outer surface"). That is, the electrolyte layers 13 are formed by laminating two or more different types of electrolyte layers 13. The two electrolyte layers 13 are in close contact with each other outside the substrate 11 and are in close contact with each other in an opening 14 provided in the substrate 11, thereby maintaining the integrity.

In the present embodiment, the substrate 11 can be formed using a resin sheet of PET or the like, and the electrode layer 12 can be laminated and formed by applying or printing a material having conductivity to the substrate 11. The substrate 11 can of course be provided with a conductive layer for guiding a biosignal acquired by the electrode layer 12 to a terminal portion (not illustrated), and this conductive layer can also be laminated and formed by applying or printing a conductive material to the substrate 11. The substrate 11 may also be provided with a shield layer made of a conductive material for blocking an electric signal or an electromagnetic wave from the outside after securing electrical insulation between the electrode layer 12 and the conductive layer.

As illustrated in Figs. 1(A) and 1(C), the opening 14 is provided in the substrate 11 in its thickness direction. In the present embodiment, while the four openings 14 having a small diameter are formed, it is also possible to form, without limiting thereto, the openings 14 having a smaller opening diameter or the openings 14 having a larger opening diameter. In addition, a region of the substrate 11 where the electrode layer 12 and the conductive layer are not formed may be formed in a mesh shape.

Thus, by using the substrate 11 having the openings 14 formed thereon, the first electrolyte layer 13a that is in close contact with the living body and is swollen by absorbing a body fluid such as sweat exuded from the living body can release the moisture from the openings 14 to the second electrolyte layer 13b, so that the moisture of the living-body contact surface in the electrolyte can be appropriately maintained. It is noted that the first electrolyte layer 13a and the second electrolyte layer 13b may be formed using different gels as described above, and also both may be formed using the same gel.

The electrolyte layers 13 are covered with a cloth 16 in the present embodiment. The cloth 16 can be formed using various types of fabrics such as a woven fabric, a knitted fabric, and a nonwoven fabric, and is desirably provided so as to cover the outer surface of the electrolyte layers 13. By providing the cloth 16, it is possible to suppress leakage of the electrolyte from an opening of a cover member 17 described below.

The cover member 17 for fixing the electrode portion 10 to the skin of the living body is provided on an outer surface of the cloth 16. The cover member 17 in the present embodiment is formed of a tape having an adhesive or pressure-sensitive adhesive layer, so that the electrode portion 10 can be stuck to the living body. However, in a case where the electrode portion 10 is fixed to the skin of the living body using another structure or material, it is not necessary to use the tape for the cover member 17, and the cover member 17 may be formed using a sheet for covering only the electrode portion 10 and suppressing the leakage of the electrolyte. Particularly when the electrode portion 10 is stuck to the living body with the cover member 17, the cover member 17 is desirably formed of a material having air permeability. Thus, the problem of stuffiness (itchiness) in the stuck portion can be solved.

An opening 15 for releasing excess moisture accumulated in the electrolyte layers 13 is formed in the cover member 17. The opening 15 is formed so as to penetrate the cover member 17 in its thickness direction in a region covering the electrolyte layers 13, and is formed in a curved elongated elliptical shape at a position facing the electrolyte layers 13 in the present embodiment. The opening 15 can be formed in various shapes and sizes as long as the opening 15 can release moisture in the electrolyte and prevent falling off of the electrolyte, and may also be formed as a fine flow path or a ventilation path. Therefore, the cover member 17 may also be formed of a material having air permeability or water permeability. In this case, the flow path or the ventilation path functions as the opening.

The opening 15 formed in the cover member 17 is desirably formed at a position not facing the openings 14 formed in the substrate 11 (that is, a position shifted in a width direction of the electrolyte layers 13). This is because there is a risk of outflow of the electrolyte due to an increase in moisture transfer at a position where both of the openings 14 and 15 face each other.

A sheet member 18 that openably closes the opening 15 is provided on an outer surface (an upper surface in the drawing) of the cover member 17. The sheet member 18 can be provided on the cover member 17 with an adhesive or the like, or can also be integrated with the cover member 17 by welding or the like. The sheet member 18 is stretched so as to cover the opening 15 of the cover member 17, so that it is possible to prevent moisture from entering the electrolyte layers 13 from the outside as much as possible, and to achieve moisture release from the electrolyte layers 13. That is, the sheet member 18 can function as a check valve related to the moisture transfer, and thus is desirably formed of a flexible sheet.

In the electrode portion 10 formed as described above, a body fluid such as sweat exuded from the living body enters the first electrolyte layer 13a, and transfers to the second electrolyte layer 13b through the openings 14 provided in the substrate 11 and a joining surface between the electrolytes outside the substrate 11. The body fluid passes through the cloth 16 covering the second electrolyte layer 13b from the second electrolyte layer 13b to be released to the outside from the opening 15 formed in the cover member 17. As a result, it is possible to exhibit an unprecedented effect of suppressing the stuffiness of the skin of a wearer. On the other hand, regarding the moisture entry from the outside, the opening 15 of the cover member 17 and the sheet member 18 that closes the opening 15 can function as a check valve, and the second electrolyte layer 13b can also function to prevent the moisture entry into the first electrolyte layer 13a. That is, when the wearer takes a bath while wearing the bioelectrode, a small amount of water may enter from the opening 15 of the cover member 17. However, the second electrolyte layer 13b can function to prevent the water entry. This is because when the second electrolyte layer 13b absorbs moisture and becomes saturated, the second electrolyte layer 13b does not absorb moisture any more, and can thereby prevent the water entry. The leakage of the first electrolyte layer 13a can be suppressed by the second electrolyte layer 13b, the cloth 16, and the sheet member 18, and a decrease in the electrolyte layers 13 due to long-term use and adhesion to clothing at the time of wearing can be suppressed.

Fig. 3 is a longitudinal cross-sectional view illustrating a bioelectrode according to another embodiment. The bioelectrode illustrated in this drawing is configured as an electrode part having a laminate structure in which a hydrophilic gel layer 13d made of a hydrophilic gel such as urethane is sandwiched between water-absorbent gel layers 13c made of a water-absorbent gel such as acryl. That is, the bioelectrode is formed as a structure in which the water-absorbent gel layer 13c (first-layer electrolyte layer), the hydrophilic gel layer 13d (second-layer electrolyte layer), and the water-absorbent gel layer 13c (third-layer electrolyte layer) are laminated from the living body side. The substrate 11 provided with the openings 14 is present between the water-absorbent gel layer 13c (first-layer electrolyte layer) and the hydrophilic gel layer 13d (second-layer electrolyte layer). According to this bioelectrode, moisture can phase-transfer between the water-absorbent gel layers 13c and the hydrophilic gel layer 13d. When the water-absorbent gel layers 13c excessively absorb moisture, the moisture can be phase-transferred to the hydrophilic gel layer 13d, and when the water-absorbent gel layers 13c become dry and lack moisture, the moisture of the hydrophilic gel layer 13d can be supplied. That is, by providing the hydrophilic gel layer 13d, the water content of the water-absorbent gel layers 13c can be adjusted. It is noted that, in the present embodiment, the bioelectrode can be configured as a bioelectrode such that the hydrophilic gel layer 13d made of a hydrophilic gel is provided in the water-absorbent gel layers 13c made of a water-absorbent gel, that is, can have a structure in which the hydrophilic gel layer 13d is sandwiched between the water-absorbent gel layers 13c.

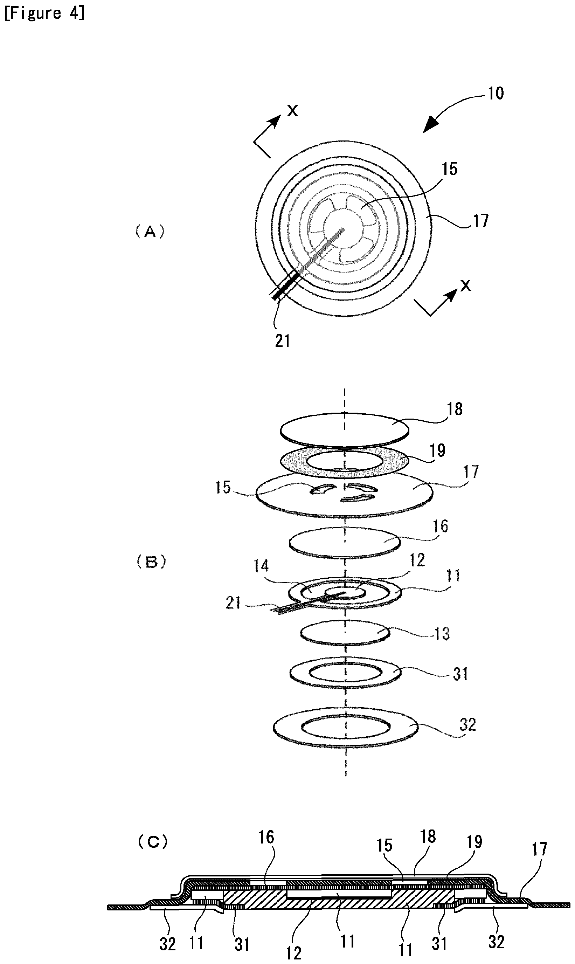

Fig. 4(A) is a plan view, Fig. 4(B) is an exploded perspective view, and Fig. 4(C) is a cross-sectional view taken along line X-X of an electrode portion 10 in a bioelectrode according to still another embodiment. In the electrode portion 10 illustrated in this drawing, a substrate 11 on which an electrode layer 12 and a conductive layer 21 are laminated is provided with an opening 14 penetrating in a thickness direction, and a cloth 16 is provided on a surface of the substrate 11 opposite to a surface on which the electrode layer 12 and the conductive layer 21 are laminated so as to cover the opening 14. A cover member 17 having an opening 15 is provided on the cloth 16, and a sheet member 18 is provided on the cover member 17.

The opening 14 provided in the substrate 11 is formed in a region where the electrode layer 12 does not exist, and can function to transfer moisture accumulated in the electrolyte layer 13 to the cloth 16. The moisture seeping into the cloth 16 is released to the outside through the opening 15 provided in the cover member 17. Therefore, the opening 14 provided in the substrate 11 and the opening 15 provided in the cover member 17 are desirably formed facing each other.

The sheet member 18 laminated on the cover member 17 always closes the opening 15 provided in the cover member 17, and the moisture seeping into the cloth 16 can be released by the moisture permeability or air permeability of the sheet member 18 itself. That is, the sheet member 18 is bonded to the cover member 17 by applying an adhesive or pressure-sensitive adhesive 19 to the periphery thereof by pattern printing or by thermally welding the periphery thereof. At this time, the opening 15 is closed by the sheet member 18, but moisture passing through the opening 15 can be released to the outside by using a film or a sheet having excellent moisture permeability or air permeability as the sheet member 18. This makes it possible to achieve the air permeability or moisture permeability from the inside while suppressing the moisture entry from the outside. The sheet member 18 having the moisture permeability or air permeability can be formed of, for example, a foamed resin, or a resin sheet having fine through-holes.

In the present embodiment, the electrolyte layer 13 is laminated on the electrode layer 12 provided on the substrate, and a support member 31 is provided so as to surround the periphery of the electrolyte layer 13. The support member 31 can function to hold the electrolyte layer 13, and is formed so that at least a part of the electrolyte layer 13 can be brought into contact with the living body. In the present embodiment, the support member 31 is formed as an annular sheet, and an opening having a size capable of supporting a rim portion of the electrolyte layer 13 is provided at its central portion. However, the support member 31 is not limited thereto, and can be formed with a plurality of openings ubiquitous or unevenly distributed. In addition, the support member 31 can be formed using a cloth such as a nonwoven fabric, and can also be formed of a raised resin sheet or a net-like sheet.

The support member 31 is bonded to the surface of the substrate on which the electrolyte layer 13 is provided, and is provided so as to surround the periphery of the electrolyte layer 13. The support member 31 can be joined to the substrate 11 by an adhesive or a pressure-sensitive adhesive, and it is desirable to provide an adhesive or a pressure-sensitive adhesive in a region of the support member 31 in contact with the living body as well. An annular holding member 32 is provided on the surface on the living body side of the support member 31 to hold the support member 31 and bring the electrode portion into close contact with the living body. Therefore, in order for the holding member 32 to be in close contact with the living body and to be joined to the support member and the cover member 17, an adhesive or a pressure-sensitive adhesive can be provided on both surfaces of the holding member 32, or the holding member 32 can be joined by thermal welding.

The support member 31 may be formed by extending and folding an end of the cloth 16 provided so as to cover the opening 14 of the substrate 11. That is, the support member 31 may be formed by folding back the end of the cloth 16 laminated on the upper side of the substrate 11 to the lower side of the substrate 11 such that the electrolyte layer 13 is held on the distal end side of the folded portion. In this case, a part of the contour of the substrate is desirably formed in a linear shape so that the cloth 16 can be folded back.

The support member 31 provided as described above can prevent deformation and movement of the electrolyte layer 13 even when the electrolyte layer 13 is swollen by absorbing moisture. Therefore, the electrolyte layer 13 can be held on the electrode layer 12 or the living body surface even when the bioelectrode is worn for a long time, and the problem that measurement becomes unstable or impossible due to falling off of the electrolyte layer 13 can be solved.

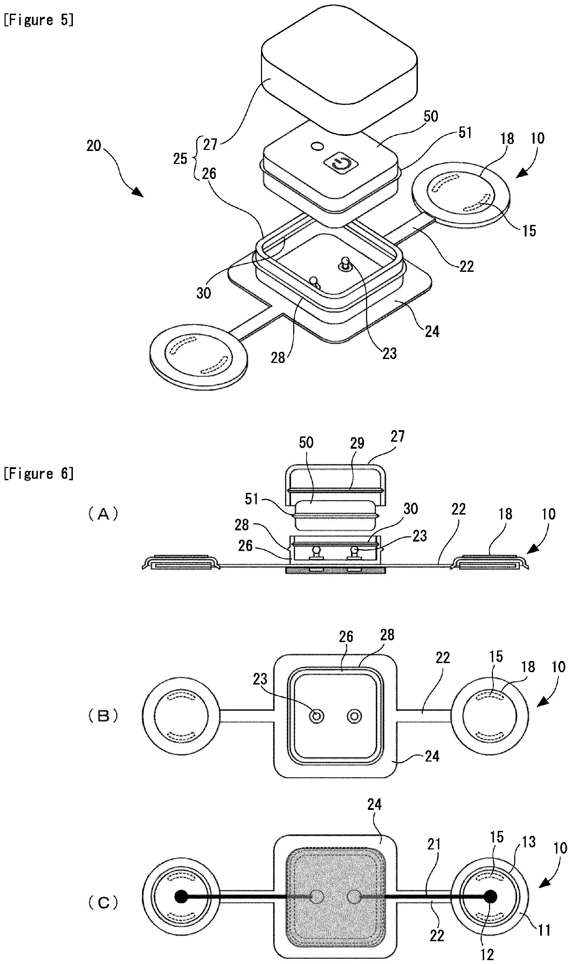

The bioelectrode including the electrode portion 10 can further include a connector or the like for transmitting and receiving signals to and from the biosignal processor 50. Such a connector is formed in a snap shape as illustrated in Figs. 5 and 6 described below, or may also be formed as a terminal formed by applying a conductive material to the substrate 11, or may be formed in other existing terminal shapes. In addition, the number of electrode parts that can be provided in one bioelectrode may be two, or may be three or more.

Hereinafter, an example of the bioelectrode including the electrode portion 10 will be described with reference to Figs. 5 and 6. Fig. 5 is a perspective view illustrating the bioelectrode using the electrode portion 10. Fig. 6(A) illustrates a longitudinal cross-sectional view of the bioelectrode, Fig. 6(B) illustrates a plan view of the bioelectrode in a state where a lid member 27 is removed, and Fig. 6(C) illustrates a bottom view of the bioelectrode. That is, the electrode portion 10 having the above structure can be formed as a bioelectrode including the waterproof member 25 as illustrated in Figs. 5 and 6. It is needless to say that the electrode portion 10 can be formed as a bioelectrode without the waterproof member 25.

As illustrated in Figs. 5 and 6, the bioelectrode according to the present embodiment includes the electrode portion 10 described above, and also includes a conductive portion 22 provided with the conductive layer 21 that transmits a signal acquired by the electrode portion 10, a snap portion 23 that outputs the biosignal guided by the conductive portion 22 to the outside, a connection portion 24 provided with the snap portion 23, and the waterproof member 25 provided in the connection portion. The electrode portion 10, the conductive portion 22, and the connection portion 24 can be laminated and formed by applying or printing the electrode layer 12 and the conductive layer 21 made of a conductive material to the substrate 11 formed of continuous sheets. However, a portion that outputs the biosignal guided by the conductive portion 22 to the outside is not necessarily the snap portion 23, and can be formed as various connectors including a waterproof connector.

In the present embodiment, in order to define a sealed space for housing the biosignal processor 50 including a terminal portion connected to the snap portion 23, the waterproof member 25 is formed of a waterproof member main body 26 serving as a lower container and the lid member 27 engaged with the water release member main body. The biosignal processor 50 is housed in the space defined by the waterproof member main body 26 and the lid member 27, and can be thereby waterproofed. Therefore, the waterproof member main body 26 and the lid member 27 need to be engaged in a state where a waterproof property is secured so that water or the like does not enter the defined internal space. In the present embodiment, a protrusion strip 28 that goes around an outer peripheral surface of the waterproof member main body 26 is formed, and a recessed groove 29 that is complementarily fitted with the protrusion strip 28 is formed on an inner surface of a side wall of the lid member 27, thereby achieving waterproofing between the waterproof member main body 26 and the lid member 27. Such a waterproof structure can be appropriately changed depending on the material constituting the waterproof member 25. An O-ring or the like may be provided in the engagement portion between the waterproof member main body 26 and the lid member 27 as necessary to achieve waterproofing.

In the present embodiment, in order to further secure waterproofing at a joining portion between a bipolar terminal portion provided in the bioelectrode and a bipolar terminal portion on the biosignal processor side, a groove 30 that receives a protrusion 51 formed on a side wall surface of the biosignal processor 50 is formed around an inner surface of a side wall of the waterproof member main body 26. By fitting the protrusion 51 of the biosignal processor 50 into the groove 30, it is possible to prevent moisture from entering the terminal portion (the snap portion 23) side relative to the groove 30. As a result, it is possible to more reliably waterproof the terminal portion. In the biosignal processor 50, the protrusion 51 may not be formed on the side wall surface in some cases. Therefore, instead of the groove, a protrusion strip that is in close contact with the side wall of the biosignal processor 50 may be formed around the inner surface of the side wall of the waterproof member main body 26.

In the case where the waterproof structure (including the groove or the protrusion strip) in close contact with the side wall surface of the biosignal processor 50 is formed on the inner surface of the side wall of the waterproof member main body 26 as described above, it is also conceivable that the terminal portion (that is, a joining portion between the connector 23 of the bioelectrode 20 and the connector of the biosignal processor 50) can be waterproofed in this region. At this time, the lid member 27 of the waterproof member 25 may be omitted on the assumption that the biosignal processor 50 is formed with a waterproof structure.

On a bottom surface of the waterproof member main body 26, the snap portion 23 provided in the connection portion 24 exists so as to be exposed. In order to prevent electrical short circuit due to water, sweat, or the like between such snap portions, it is necessary to reliably achieve waterproofing between the snap portions.
Therefore, in the present embodiment, the connection portion 24 and a bottom portion of the waterproof member main body 26 are tightly connected by crimping using the snap portions 23, thereby achieving waterproofing between the snap portions 23.

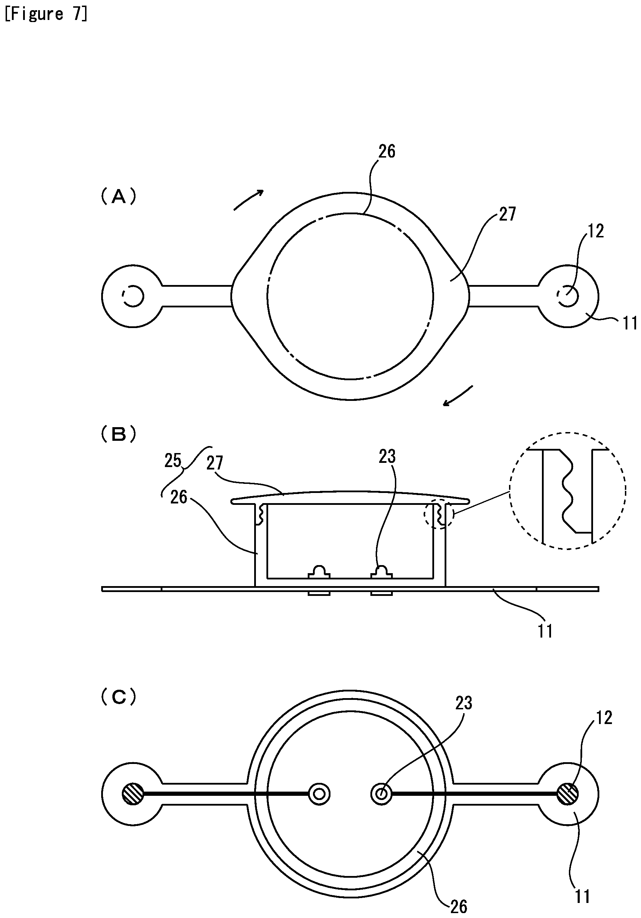

Fig. 7(A) is a plan view, Fig. 7(B) is a longitudinal cross-sectional view, and Fig. 7(C) is a bottom view illustrating a bioelectrode according to another embodiment. In particular, in the bioelectrode according to this embodiment, a waterproof member 25 achieves waterproofing of a biosignal processor connected to a snap portion (terminal portion) 23, so that the biosignal processor (not illustrated) having no waterproof function can be used.

The waterproof member 25 according to the present embodiment includes a waterproof member main body 26 having a bottomed cylindrical shape in which the snap portion (terminal portion) 23 such as two-pole snaps protrudes from a bottom surface, and a lid member 27 that closes an internal space of the waterproof member main body 26, and has a structure in which a joining portion between the waterproof member main body 26 and the lid member 27 has a waterproof property. In particular, in the present embodiment, the waterproof member main body 26 and the lid member 27 are formed so as to secure the waterproof property by a rotary lock mechanism that seals a gap between the waterproof member main body 26 and the lid member 27 by rotating the waterproof member main body 26 and the lid member 27 after combining the waterproof member main body 26 and the lid member 27. A seal member such as an O-ring may of course be provided between the waterproof member main body 26 and the lid member 27. By securing the waterproof property at an engagement portion between the waterproof member main body 26 and the lid member 27 as described above, the biosignal processor connected to the snap portion 23 does not have to have a waterproof structure, and as long as the biosignal processor can be housed in the internal space of the waterproof member, the biosignal processor may have any size and shape. Thus, the range of the biosignal processor that can be used is widened.

The coupling structure between the waterproof member main body 26 and the lid member 27 can be changed as long as the waterproof property between the waterproof member main body 26 and the lid member 27 can be secured. The coupling structure may be a screwing structure or a joining structure in addition to a fitting structure. In addition, by preparing a plurality of the lid members 27 with different heights that can be coupled to the waterproof member main body 26, any height can be selected according to the height (thickness) of the biosignal processor connected to the terminal.

As described above, the bioelectrode including the waterproof member 25 can waterproof the biosignal processor 50 as well. Therefore, it is possible to achieve the bioelectrode that is wearable even during bathing and is thus wearable for a long time. The comfort of wearing the bioelectrode because of the electrode portion 10 provided with the water discharge structure (the openings 14 and 15) also contributes to achieving the bioelectrode that is wearable for a long time.

Note that the bioelectrode including the waterproof member 25 illustrated in Figs. 5 to 7 is not limited to the electrode portion illustrated in Figs. 1 to 4, and may be formed to include various electrode parts. That is, in order to define the sealed space for housing the biosignal processor 50, the bioelectrode includes the waterproof member 25 formed of the waterproof member main body 26 as the lower container and the lid member 27 engaged with the water release member main body.

This bioelectrode is a bioelectrode including an electrode portion 10 that acquires an electric signal of a living body or outputs an electric signal to the living body, the bioelectrode including a terminal portion including two or more pole terminals that input or output the electric signal in the electrode portion 10 to the outside, the bioelectrode further including a waterproof member 25 that waterproofs at least a connection portion between the terminal portion and a terminal portion of a biosignal processor 50 in a state where the terminal portion is connected to the terminal portion of the biosignal processor 50. In the bioelectrode, the waterproof member 25 can be configured to waterproof and define a housing space that houses the biosignal processor 50.

### Industrial Applicability

The bioelectrode of the present invention can be used for acquiring biosignals or for iontophoresis, and can be used as a bioelectrode that can be used in Holter electrocardiography in which an electrocardiogram is recorded for a long time such as 24 hours or several days. Since moisture due to sweat or the like is discharged from the openings even when the bioelectrode is worn for a short time, the bioelectrode can be used as a bioelectrode that is worn comfortably even during exercise or work.

### Reference Signs List

- 10: Electrode portion
- 11: Substrate
- 12: Electrode layer
- 13: Electrolyte layer
- 13c: Water-absorbent gel layer
- 13d: Hydrophilic gel layer
- 14, 15: Opening
- 16: Cloth
- 17: Cover member
- 18: Sheet member
- 20: Bioelectrode
- 21: Conductive layer
- 22: Conductive portion
- 23: Snap portion (terminal portion)
- 25: Waterproof member
- 26: Waterproof member main body
- 27: Lid member
- 50: Biosignal processor

## Claims

1. A bioelectrode comprising an electrode portion that acquires an electric signal of a living body or outputs an electric signal to the living body, wherein
the electrode portion is provided with an electrolyte layer that is in close contact with the living body,
the electrode portion is provided with a sheet-like cover member that covers at least a part or all of a surface of the electrolyte layer on a side not in close contact with the living body, and
an opening that penetrates in a thickness direction is provided in a region of the cover member covering the electrolyte layer.

2. A bioelectrode comprising an electrode portion that acquires an electric signal of a living body or outputs an electric signal to the living body, wherein
the electrode portion is formed by laminating a conductive material on a substrate,
an electrolyte layer that is in close contact with the living body is provided on a surface of the substrate on a side where the conductive material is laminated, and
the substrate is provided with an opening that penetrates in a thickness direction.

3. The bioelectrode according to claim 1 or 2, wherein the opening is closed by a member having water permeability or water absorbency, the member formed of a hydrophilic gel, a water-absorbent gel, a hydrophobic gel, a cloth, or a porous sheet, or is openably closed by a sheet member.

4. A bioelectrode comprising an electrode portion that acquires an electric signal of a living body or outputs an electric signal to the living body, wherein
the electrode portion is provided with an electrolyte layer that is in close contact with the living body, and
at least the electrolyte layer is formed by laminating a water-absorbent gel layer formed of a gel having water absorbency and a hydrophilic gel layer formed of a gel having hydrophilicity in a part or all of a region.

5. The bioelectrode according to any one of claims 1, 2, and 4, wherein the substrate is provided with a support member that supports a rim of the electrolyte layer in a state where at least a part of the electrolyte layer is exposed to the living body side.

6. A bioelectrode comprising an electrode portion that acquires an electric signal of a living body or outputs an electric signal to the living body,
the bioelectrode comprising: a terminal portion including two or more pole terminals that input or output the electric signal in the electrode portion to an outside,
the bioelectrode further comprising a waterproof member that waterproofs, in a state where the terminal portion is connected to a terminal portion of a biosignal processor, a connection portion at least between the terminal portions.

7. The bioelectrode according to claim 6, wherein the waterproof member waterproofs and defines a housing space that houses the biosignal processor.

## Amended claims

### Amended claims under Art. 19.1 PCT

1. (Amended) A bioelectrode comprising an electrode portion that acquires an electric signal of a living body or outputs an electric signal to the living body, wherein
the electrode portion is provided with an electrolyte layer that is in close contact with the living body,
the electrode portion is provided with a sheet-like cover member that covers at least a part or all of a surface of the electrolyte layer on a side not in close contact with the living body,
an opening that penetrates in a thickness direction is provided in a region of the cover member covering the electrolyte layer, and
a sheet member that openably closes the opening is provided on an outer surface of the cover member.

2. (Amended) A bioelectrode comprising an electrode portion that acquires an electric signal of a living body or outputs an electric signal to the living body, wherein
the electrode portion is formed by laminating a conductive material on a substrate,
an electrolyte layer that is in close contact with the living body is provided on a surface of the substrate on a side where the conductive material is laminated, and
the substrate is provided with an opening that penetrates in a thickness direction in a region of the substrate where the electrode layer and a conductive layer are not formed.

3. The bioelectrode according to claim 1 or 2, wherein the opening is closed by a member having water permeability or water absorbency, the member formed of a hydrophilic gel, a water-absorbent gel, a hydrophobic gel, a cloth, or a porous sheet, or is openably closed by a sheet member.

4. A bioelectrode comprising an electrode portion that acquires an electric signal of a living body or outputs an electric signal to the living body, wherein
the electrode portion is provided with an electrolyte layer that is in close contact with the living body, and
at least the electrolyte layer is formed by laminating a water-absorbent gel layer formed of a gel having water absorbency and a hydrophilic gel layer formed of a gel having hydrophilicity in a part or all of a region.

5. (Amended) The bioelectrode according to any one of claims 1, 2, and 4, wherein the substrate is provided with a support member that supports a rim of the electrolyte layer in a state where at least a part of the electrolyte layer is exposed to the living body side.
26

6. (Amended) A bioelectrode comprising an electrode portion that acquires an electric signal of a living body or outputs an electric signal to the living body,
the bioelectrode comprising a terminal portion including two or more pole terminals that input or output the electric signal in the electrode portion to an outside,
the bioelectrode further comprising a waterproof member that waterproofs, in a state where the terminal portion is connected to a terminal portion of the biosignal processor, a connection portion at least between the terminal portions,
wherein the waterproof member includes a housing space for the biosignal processor, and the terminal portions exist in the housing space.

7. The bioelectrode according to claim 6, wherein the waterproof member waterproofs and defines a housing space that houses the biosignal processor.

Statement under Art. 19.1 PCT
Claim 1 clarifies that "a sheet member that openably closes the opening is provided on an outer surface of the cover member" based on the description in the paragraph number [0058] column of the present specification.

On the other hand, in D1 (US 5431166 A) described in the International Search Report, the central aperture 36 provided so as to expose the tab 30 where the connector 40 is installed remains open and is not closed at all.

In the present invention, the opening provided in the cover member is for releasing moisture of the electrolyte layer covered by the cover member to the outside, and is not for installing the connector 40 in D1.

Therefore, the configuration of "a sheet member that openably closes the opening is provided on an outer surface of the cover member" in amended claim 1 is unpredictable from D1.

Furthermore, based on the description in the paragraph number [0052] column of the present specification, claim 2 clarifies that the opening that penetrates in the thickness direction is provided in the substrate "in a region of the substrate where the electrode layer and a conductive layer are not formed".

On the other hand, in D1 (US 5431166 A) described in the International Search Report, the central aperture 36 is provided in the annular retaining sheet 34, not in the substrate (the base sheet 24). This substrate sheet is not provided with any openings.

Therefore, the configuration in which the opening is provided "in a region of the substrate where the electrode layer and a conductive layer are not formed" in amended claim 2 is unpredictable from D1.

Based on the description in the paragraph number [0071], [0074], and [0076] columns of the present specification, and Figs. 6 and 7, claim 6 clarifies that "the waterproof member includes a housing space for the biosignal processor, and the terminal portions exist in the housing space".

On the other hand, in Cited Document 3 (WO 2018/220729), the connection between the bioelectrode 2 and the module 3 is achieved by the inner element 32 and the outer element 31, which is the outer side of the module cover 25 accommodating the module.

Therefore, it is unpredictable from D3 that "the waterproof member includes a housing space for the biosignal processor, and the terminal portions exist in the housing space" in amended claim 6.
